(19) Europäisches Patentamt
European Patent Office
Office européen des brevets

(11) Veröffentlichungsnummer : **0 065 692**
**B1**

# (12) EUROPÄISCHE PATENTSCHRIFT

(45) Veröffentlichungstag der Patentschrift : **19.12.84**

(51) Int. Cl.³ : **C 07 C 93/06**, A 61 K 31/13

(21) Anmeldenummer : **82104047.4**

(22) Anmeldetag : **10.05.82**

(54) **Erythro-1,2,3-Triphenyl-1-pentanon-Derivate sowie Verfahren zu ihrer Herstellung und ihre Verwendung als Arzneimittel.**

(30) Priorität : **27.05.81 DE 3121175**

(43) Veröffentlichungstag der Anmeldung : **01.12.82 Patentblatt 82/48**

(45) Bekanntmachung des Hinweises auf die Patenterteilung : **19.12.84 Patentblatt 84/51**

(84) Benannte Vertragsstaaten : **AT BE CH DE FR GB IT LI LU NL SE**

(56) Entgegenhaltungen :
**FR-A- 2 329 264**
**US-A- 3 494 934**
**CHEMICAL ABSTRACTS, Band 74, Nr. 9, 1. März 1971, Seite 233, Nr. 40891a, Columbus Ohio (USA); R. GOPALCHARI et al.: "Antifertility agents. V. 3-Alkyl-2,3-diphenylpropiophenones"**
**CHEMICAL ABSTRACTS, Band 79, Nr. 11, 17. September 1973, Seite 11, Nr. 61404n, Columbus Ohio (USA); R. GOPALCHARI et al.: "Antifertility agents. Synthesis of dialkylaminoethoxy derivatives of 3-alkyl-2,3-diphenylpropiophenones"**

(73) Patentinhaber : **Klinge Pharma GmbH**
**Berg-am-Laim-Strasse 129**
**D-8000 München 80 (DE)**

(72) Erfinder : **Schickaneder, Helmut, Dr.**
**Moosäcker 25**
**D-8501 Eckental-Eckenhaid (DE)**
Erfinder : **Löser, Roland, Dr.**
**Fichtenweg 2**
**D-8133 Feldafing (DE)**

(74) Vertreter : **Wuesthoff, Franz, Dr.-Ing. et al**
**Patentanwälte Wuesthoff -v. Pechmann-Behrens-Goetz Schweigerstrasse 2**
**D-8000 München 90 (DE)**

**EP 0 065 692 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung bezieht sich auf in bestimmter Weise substituierte basische Erythro-1,2,3-triphenyl-1-pentanon-derivate und deren verträgliche Salze, welche verbesserte therapeutische Eigenschaften besitzen.

In ihrer Grundstruktur können diese Verbindungen auch als basische Erythro-3-alkyl-2,3-diphenyl-propiophenone betrachtet werden. Unter dieser Bezeichnung sind in « Contraception » *2,* 199-205 (1970) und « Indian J. Chem. » *11,* 229-233 (1973) einige Verbindungen beschrieben worden, die eine Antifertilitäts- und Antiimplantationsaktivität besitzen. Von den beschriebenen Substanzen zählt das Erythro-1-[4'-(2-pyrrolidin-1-yl-ethoxy)phenyl]-2,3-diphenyl-1-pentanon [Verbindung III in « Contraception » bzw. Verbindung IV in « Indian J. Chem. »] und das Erythro-1-[4'-(2-Dimethylaminoethoxy)phenyl]-2-phenyl-3-(4'-chlorophenyl)-1-pentanon [Verbindung X in « Indian J. Chem. »] zu den Verbindungen mit höchster Aktivität.

Wie im Vergleich zum bekannten Erythro-1-[4'-(2-pyrrolidin-1-yl-ethoxy)phenyl]-2,3-diphenyl-1-pentanon gezeigt werden konnte, sind die beanspruchten Erythro-1,2,3-triphenyl-1-pentanon-derivate sowohl in ihrer Bindungsaffinität zum Estrogenrezeptor als auch in ihrer antiuterotropen Aktivität eindeutig überlegen. Diese Eigenschaften sind für eine mammatumorhemmende Wirkung von entscheidender Bedeutung.

Die erfindungsgemäßen Erythro-1,2,3-triphenyl-1-pentanonderivate entsprechen der allgemeinen Formel

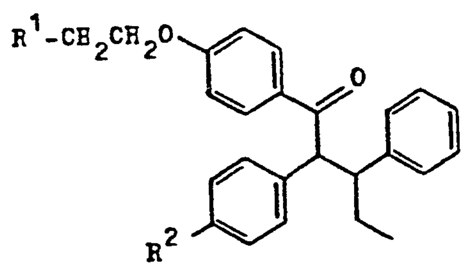

(1)

in der $R^1$ eine Dimethylamino- oder Diethylaminogruppe sein kann und $R^2$ eine Methoxy- oder Hydroxygruppe darstellt.

Die Erfindung betrifft ferner ein Verfahren zur Herstellung von Verbindungen der allgemeinen Formel (1), das dadurch gekennzeichnet ist, daß man 1,2-Diphenylethanone der allgemeinen Formel

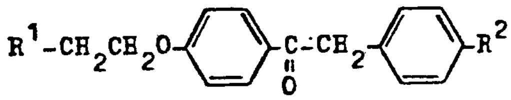

(2)

in der $R^1$ die in der allgemeinen Formel (1) angegebene Bedeutung besitzt und $R^2$ eine Methoxygruppe darstellt, in wasserfreiem Dimethylformamid mit Natriumhydrid reagieren läßt und nach Umsetzung mit 1-Chlor-1-phenylpropan die Erythroform durch Kristallisation isoliert. Die Verbindungen der allgemeinen Formel (1), in der $R^2$ eine Hydroxygruppe darstellt, können dann in einer 2. Verfahrensstufe dadurch erhalten werden, daß man die Methoxyverbindungen der allgemeinen Formel (1) einer an sich bekannten selektiven Methyletherspaltung mit Bromwasserstoff unterzieht.

Die erfindungsgemäßen Verbindungen lassen sich beispielsweise wie folgt darstellten :

Zunächst werden Verbindungen der allgemeinen Formel

$$R^1—CH_2CH_2Cl \qquad (3)$$

in der $R^1$ eine Dimethylamino- oder Diethylaminogruppe sein kann, in Gegenwart von Alkali mit Phenol zu Ethern der allgemeinen Formel

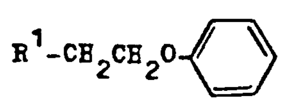

(4)

in der $R^1$ die oben angegebene Bedeutung besitzt, umgesetzt. Diese Phenolether lassen sich in einer Friedel-Crafts-Reaktion mit Phenylessigsäurechloriden der allgemeinen Formel

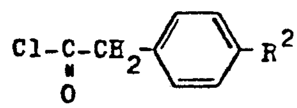

(5)

in der $R^2$ eine Methoxygruppe darstellt, zu den 1,2-Diphenylethanonen der allgemeinen Formel (2) umsetzen. Nach Einwirken von Natriumhydrid und anschließender Umsetzung mit 1-Chlor-1-phenyl propan werden in Gegenwart von wasserfreien Dimethylformamid die erfindungsgemäßen Verbindungen der allgemeinen Formel (1) erhalten, in der $R^2$ eine Methoxygruppe darstellt.

Durch Kristallisation aus verdünntem Methanol fallen die Verbindungen in ihrer reinen Erythroform an.

Die Methoxyderivate der allgemeinen Formel (1) können mit Bromwasserstofflösung unter Rückfluß erhitzt und durch Abspaltung der Methylgruppe in die Verbindungen der allgemeinen Formel (1), in denen $R^2$ eine Hydroxygruppe darstellt, umgewandelt werden.

Die nachfolgenden Beispiele erläutern die Herstellung erfindungsgemäßer Verbindungen.

## Beispiel 1

erythro-1-[4'-(2-Dimethylaminoethoxy)-phenyl]-2-(4'-methoxyphenyl)-3-phenyl-1-pentanon

### Herstellung der Vorstufen

1-[4'-(2-Dimethylaminoethoxy)-phenyl]-2-(4'-methoxyphenyl)-1-ethanon

20,2 g (0,1 mol) N,N-Dimethyl-2-phenoxyethylamin-hydrochlorid und 18,5 g (0,1 mol) 4-Methoxyphenylessigsäurechlorid werden in 350 ml wasserfreiem Methylenchlorid suspendiert und bei Raumtemperatur mit 26,7 g (0,2 mol) wasserfreiem Aluminiumchlorid portionsweise versetzt. Nach beendeter Zugabe wird die Reaktionslösung 1 Stunde auf Rückflußtemperatur erhitzt, anschließend auf Eis gegossen und mit 25 %iger Natronlauge alkalisch gestellt. Die organische Phase wird abgetrennt, neutral gewaschen und über Natriumsulfat getrocknet. Das Lösungsmittel wird i. Vak. entfernt und der Rückstand aus Petrolether kristallisiert, wodurch das 1-[4'-(2-Dimethylaminoethoxy)-phenyl]-2-(4'-methoxyphenyl)-1-ethanon (Schmp. 75 bis 77 °C aus Petrolether) erhalten wird.

### Herstellung der erfindungsgemäßen Verbindung

Zu einer Suspension aus 150 ml wasserfreiem Dimethylformamid und 2,4 g (0,1 mol) Natriumhydrid wird dann bei 20 °C unter $N_2$ eine Lösung von 0,1 mol des 1-[4'-(2-Dimethylaminoethoxy)-phenyl]-2-(4'-methoxyphenyl)-1-ethanons in 150 ml wasserfreiem Dimethylformamid langsam hinzugetropft. Nach beendeter Zugabe läßt man eine halbe Stunde bei Raumtemperatur nach reagieren und tropft dann eine Lösung von 18,5 g (0,12 mol) 1-Chlor-1-phenylpropan in 50 ml wasserfreiem Dimethylformamid bei Raumtemperatur zu. Nach 2 Stunden wird die Reaktionslösung mit Wasser zersetzt und in Ethylacetat aufgenommen. Die organische Phase wird mit Wasser neutralgewaschen, über Natriumsulfat getrocknet und i. Vak. eingeengt. Aus dem Rückstand läßt sich mit Methanol/Wasser die erythro-Form kristallisieren.

Es werden farblose Kristalle vom Schmp. 88 bis 89 °C (Petrolether) erhalten.

$R_f$ 0,25 [$CHCl_3$/MeOH (9/1)] ; Ausbeute : 5,6 g (13 %)

$C_{28}H_{33}NO_3$ (431,6)

Ber. : C 77,93 H 7,71 N 3,25

Gef. : C 77,92 H 7,65 N 3,18

Mol.-Gew. : 431 (massenspektrometrisch bestimmt)

IR-Spektrum (KBr) : $\nu$ (C=O) 1 665 $cm^{-1}$

$^1H$-NMR-Spektrum ($d_6$-Aceton) :

| | | | | |
|---|---|---|---|---|
| 0,58 | t | (3) | $C\underline{H}_3$ | [J = 6,0] |
| 1,10 bis 1,73 | m | (2) | $C\underline{H}_2$ | |
| 2,20 | s | (6) | $N(C\underline{H}_3)_2$ | |
| 2,60 | t | (2) | $C\underline{H}_2$—N | [J = 5,6] |
| 3,13 bis 3,73 | m | (1) | $C\underline{H}$ | |
| 3,74 | d | (3) | $OC\underline{H}_3$ | |
| 4,07 | t | (2) | $OC\underline{H}_2$ | [J = 5,6] |
| 5,20 | d | (1) | $C\underline{H}$—C=O | [J = 11,6] |
| 6,70 bis 8,13 | m | (13) | Aromaten-H | |

## Beispiel 2

erythro-1-[4'-(2-Diethylaminoethoxy)-phenyl]-2-(4'-methoxyphenyl)-3-phenyl-1-pentanon

34,1 g (0,1 mol) analog Beispiel 1 hergestelltes 1-[4'-(2-Diethylaminoethoxy)-phenyl]-2-(4'-methoxyphenyl)-1-ethanon (Schmp. 52 bis 53 °C aus Petrolether) werden dann, wie unter Beispiel 1 weiter beschrieben, umgesetzt. Man erhält farblose Kristalle vom Schmp. 89 bis 90 °C (Methanol/Wasser) ;

$R_f$ 0,35 [$CHCl_3$/MeOH (9/1)] ; Ausbeute : 3,2 g (7 %).

$C_{30}H_{37}NO_3$ (459,6)

| | | | |
|---|---|---|---|
| Ber. : | C 78,40 | H 8,11 | N 3,05 |
| Gef. : | C 78,22 | H 8,07 | N 2,92 |

Mol.-Gew. 459 (massenspektrometrisch bestimmt)

IR-Spektrum (KBr) : $\nu$ (C=O) 1 660 $cm^{-1}$

$^1$H-NMR-Spektrum ($CDCl_3$) :

| | | | | |
|---|---|---|---|---|
| 0,57 | t | (3) | $CH_3$ | [J = 6,2] |
| 1,00 | t | (6) | $CH_3$ | [J = 7,0] |
| 1,13 bis 1,73 | m | (2) | $CH_2$ | |
| 2,57 | q | (4) | $CH_2$ | [J = 7,0] |
| 2,77 | t | (2) | $CH_2$—N | [J = 5,0] |
| 3,03 bis 3,67 | m | (1) | CH | |
| 3,73 | s | (3) | $OCH_3$ | |
| 3,97 | t | (2) | $OCH_2$ | [J = 5,0] |
| 4,77 | d | (1) | CH—C = O | [J = 11,8] |
| 6,53 bis 7,90 | m | (13) | Aromaten-H | |

## Beispiel 3

erythro-1-[4'-(2-Dimethylaminoethoxy)-phenyl]-2-(4'-hydroxyphenyl)-3-phenyl-1-pentanon

43,2 g (0,1 mol) erythro-1-[4'-(Dimethylaminoethoxy)-phenyl]-2-(4'-methoxyphenyl)-3-phenyl-1-pentanon werden mit 400 ml 48 %iger, wäßriger Bromwasserstofflösung 2 Stunden unter Rückfluß erhitzt. Anschließend wird i. Vak. zur Trockne eingeengt, der Rückstand mit ca. 200 ml verdünnter wäßriger Ammoniaklösung basisch gestellt und dreimal mit je 100 ml Ethylacetat extrahiert. Die organische Phase wird mit Wasser neutral gewaschen und nach dem Trocknen über Natriumsulfat das Lösungsmittel i. Vak. entfernt. Der Rückstand wird aus Methanol mehrmals kristallisiert. Es werden farblose Kristalle vom Schmp. 200 °C erhalten.

$R_f$ 0,20 [$CHCl_3$/$CH_3OH$ (7/3)] ; Ausbeute : 24,6 g (59 %).

$C_{27}H_{31}NO_3$ (417,5)

| | | | |
|---|---|---|---|
| Ber. : | C 77,67 | H 7,48 | N 3,35 |
| Gef. : | C 77,66 | H 7,51 | N 3,23 |

Mol.-Gew. : 417 (massenspektrometrisch bestimmt)

IR-Spektrum (KBr) : $\nu$ (O—H) 3 600 bis 3 100 $cm^{-1}$

(C=O) 1 670 $cm^{-1}$

$^1$H-NMR-Spektrum ($d_6$-Aceton) :

| | | | | |
|---|---|---|---|---|
| 0,57 | t | (3) | $CH_3$ | [J = 6,0] |
| 1,17 bis 1,67 | m | (2) | $CH_2$ | |
| 2,20 | s | (6) | $N(CH_3)_2$ | |
| 2,63 | t | (2) | $CH_2N$ | [J = 5,0] |
| 2,83 bis 3,67 | m | (1) | CH | [J = 5,0] |
| 4,10 | t | (2) | $OCH_2$ | [J = 5,0] |
| 5,17 | d | (1) | CH—C=O | [J = 11,8] |
| 6,67 bis 8,17 | m | (13) | Aromaten-H | |

Die Erythro- und Threoformen der 1,2,3-Triphenyl-1-pentanonderivate der allgemeinen Formel (1) unterscheiden sich deutlich in ihren Protonenresonanzsignalen der Methylprotonen in der Pentanonkette. Die Signale der Erythroform sind bei den beanspruchten Verbindungen gegenüber der Threoform hochfeldverschoben, wie dies auch bei Hexestrol-Derivaten festgestellt wurde [R. Goswami, S. G. Harsy, D. F. Heiman und J. A. Katzenellenbogen, J. med. Chem. *23,* 1002 (1980)].

Bei den Erythroformen der 1,2,3-Triphenyl-1-pentanon-Derivate der allgemeinen Formel (1) konnte eine hohe antiestrogene Wirkung festgestellt werden, die therapeutisch nutzbar ist.

Die Bestimmung der Bindungsaffinität zum Estradiolrezeptor wurde mit dem Kaninchenuterus-Cytosol vorgenommen. Die beanspruchten Verbindungen zeigten eine hohe Bindungsaffinität.

Die Messung der antiuterotropen Wirkung erfolgte nach dreiwöchiger Wirkstoffbehandlung von geschlechtsreifen, weiblichen Ratten. Die beanspruchten Verbindungen zeigten eine ausgeprägte antiuterotrope Wirkung.

Die tumorhemmende Wirkung wurde nach vierwöchiger Wirkstoffbehandlung an weiblichen Ratten gemessen, deren Mammatumor vorher durch 7,12-Dimethylbenz(a)anthracen ausgelöst wurde. Die ausgewählten Verbindungen führten zu einer starken Hemmung des Tumorwachstums.

Die erfindungsgemäßen Verbindungen stellen somit eine wertvolle Bereicherung des Arzneimittelschatzes dar und können zur Behandlung des malignen Mammatumors eingesetzt werden.

## Pharmakologische Untersuchungen

a) Bindungsaffinität zum Estradiolrezeptor

Die Messung der Bindungsaffinität zum Estradiolrezeptor erfolgte nach der Methode von N. Devleeschouwer, G. Leclercq, A. Danguy and J. C. Heuson [Europ. J. Cancer, *14,* 721-723 (1978)]. Das Uteruscytosol von weiblichen, präpubertären, weißen 2 kg schweren Kaninchen (Neuseeländer) wurde 18 Stunden bei 40 °C mit 2,5 × $10^{-9}$ M [$^3$H]-Estradiol sowie jeweils unter Zusatz von unmarkiertem Estradiol (Kontrolle) bzw. Testsubstanz verschiedener Konzentration inkubiert. Die Bindungsaffinität zum Estradiolrezeptor wird ausgedrückt durch die, dem Uteruscytosol zugesetzte Konzentration an unmarkiertem Estradiol (Kontrolle) bzw. Testsubstanz, welche eine 50 %ige Verdrängung des am Estradiolrezeptor gebundenen [$^3$H]-Estradiol bewirkt.

Tabelle I

Bindungsaffinität der Testsubstanzen

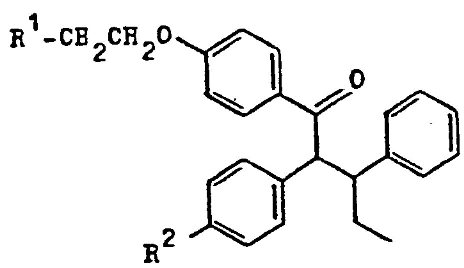

| Verbindung Nr. | $R^1$ | $R^2$ | $ED_{50}$ x) [M] |
|---|---|---|---|
| Estradiol (Kontrolle) | | | 1.3 × $10^{-9}$ |
| Vergleichs-substanz | 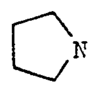 | H | 6.0 × $10^{-6}$ |
| 1 | $(CH_3)_2N$ | $OCH_3$ | 4.5 × $10^{-6}$ |
| 2 | $(C_2H_5)_2N$ | $OCH_3$ | 5.0 × $10^{-7}$ |
| 3 | $(CH_3)_2N$ | OH | 5.4 × $10^{-9}$ |

x) Konzentration der Substanz, die 50 % [$^3$H]-Estradiol vom Estradiol-Rezeptor verdrängt.

Die bei dieser Untersuchung erhaltenen Werte zeigen, daß von den beanspruchten Verbindungen die Substanz 3 die stärkste Affinität zum Estradiol-Rezeptor besitzt.

b) Antiuterotrope Wirkung

Die antiuterotrope Wirkung wurde nach einem modifizierten «Dorfman-Test» (R. I. Dorfman, Methods in Hormone Research II, S. 707, Academic Press, New York-London, 1962) an geschlechtsreifen, weiblichen Sprague-Dawley-Ratten bestimmt.

Die Testverbindungen wurden in 0,25 %iger, wäßriger Agarsuspension aufgenommen und per Schlundsonde über einen Zeitraum von 21 Tagen sechsmal wöchentlich verabreicht. Am Versuchsende wurde das uterusgewicht der mit Wirkstoff behandelten Tiere in Beziehung gesetzt zum Uterusgewicht von Kontrolltieren, die nur eine leere Agarsuspension erhielten.

Tabelle II

Antiuterotrope Aktivität der Testsubstanzen

| Verbindung No. | Zahl der Versuchstiere | Dosis mg/kg/Tag | Unterusgewicht gegenüber Kontrolltieren |
|---|---|---|---|
| Vergleichs-substanz +) | 10 | 3 | - 30% |
| 1 | 10 | 3 | - 47% |
| 3 | 10 | 3 | - 54% |

+) Als Vergleichssubstanz diente bei den Untersuchungen das Erythnol-1-[4'-(2-pyrrolidin-1-yl-ethoxy)phenyl]-2,3-diphenyl-1-pentanon, das in «Contraception» *2*, 199-205 (1970) als wirksamste Substanz vorbeschrieben war. Es besitzt deutlich geringege Aktivität als die Verbindungen der Erfindung.

c) Mammatumorhemmende Wirkung

Die tumorhemmende Wirkung wurde am Modell des mit 7,12-Dimethylbenz(a) anthracen induzierten Mammatumors der weiblichen Spraque-Dawley-Ratte (Stamm Hannover) nach der Methode von M. J. Golder [Europ. J. Cancer, *11*. 571 (1975)] und D. P. Griswold et al. [Cancer Research, *26*, 2169 (1966)] bestimmt.

Die Testverbindungen wurden in 0,25 %iger Agarsuspension aufgenommen und per Schlundsonde über einen Zeitraum von 28 Tagen sechsmal wöchentlich verabreicht. Zweimal wöchentlich und am 28. Versuchstag wurde die Zahl der Tiere festgestellt und die Tumorfläche ($mm^2$/Tier) der Therapie- und Kontrolltiere gemessen. Bestimmt wurde am Versuchsende die prozentuale Zunahme der durchschnittlichen Tumorfläche der behandelten Tiere im Vergleich zu den Kontrolltieren, die als 100 % angenommen wurde.

Tabelle III

Tumorhemmende Aktivität der Testsubstanzen

| Verbindung No. | Zahl der Versuchstiere | Dosis mg/kg/Tag | Relative Zunahme der durchschnittlichen Tumorfläche |
|---|---|---|---|
| Leerkontrolle | 10 | - | 100% |
| 3 | 10 | 3 | 30% |

**Ansprüche**

1. Erythro-1,2,3-triphenyl-1-pentanone der allgemeinen Formel

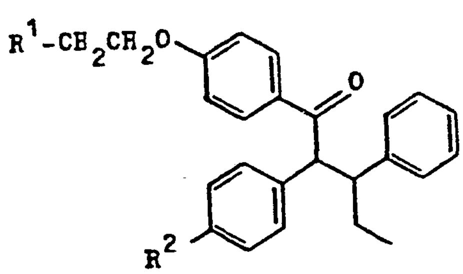

(1)

in der $R^1$ eine Dimethylamino- oder Diethylaminogruppe sein kann und $R^2$ eine Methoxy- oder Hydroxygruppe darstellt sowie deren therapeutisch verträgliche Salze.

2. Verfahren zur Herstellung von Verbindungen der allgemeinen Formel 1, dadurch gekennzeichnet, daß man 1,2-Diphenylethanone der allgemeinen Formel

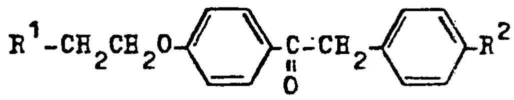

(2)

in der $R^1$ die in der allgemeinen Formel 1 angegebene Bedeutung besitzt und $R^2$ eine Methoxygruppe darstellt, in wasserfreiem Dimethylformamid mit Natriumhydrid reagieren läßt, nach Umsetzung mit 1-Chlor-1-phenylpropan die Erythroform durch Kristallisation isoliert und gegebenenfalls die Hydroxygruppe durch selektive Spaltung der Methoxygruppe mit Bromwasserstoff freisetzt.

3. Arzneimittel enthaltend eine Verbindung gemäß Anspruch 1 als Wirkstoff sowie deren übliche Träger- und Hilfsstoffe.

**Claims**

1. Erythro-1,2,3-triphenyl-1-pentanones represented by the general formula

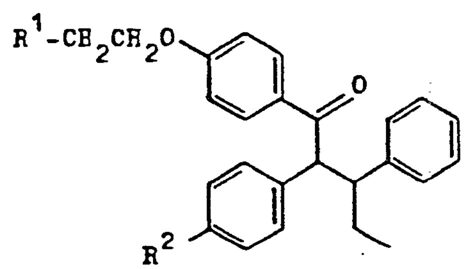

(1)

wherein $R^1$ may be a dimethylamino or diethylamino group and $R^2$ represents a methoxy or hydroxy group as well as the therapeutically acceptable salts thereof.

2. Process for the production of compounds represented by general formula 1, characterized in that 1,2-diphenylethanones represented by the general formula

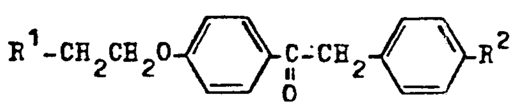

(2)

wherein $R^1$ has the meaning indicated in general formula 1 and $R^2$ represents a methoxy group, is reacted in anhydrous dimethylformamide with sodium hydride, that after the reaction with 2-chloro-1-phenylpropane the erythroform is isolated by crystallization and optionally the hydroxy group is set free by selective cleavage of the methoxy group with hydrogen bromide.

3. A pharmaceutical preparation containing a compound according to claim 1 as active component, as well as the usual carrier and auxiliary products.

**Revendications**

1. Erythro-1,2,3-triphényl-1-pentanones de formule générale

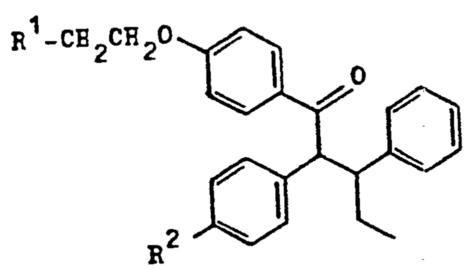

(1)

où $R^1$ peut être un groupe diméthylamino ou diéthylamino et $R^2$ représente un groupe méthoxy ou hydroxy ainsi que leurs sels thérapeutiquement acceptables.

2. Procédé de préparation de composés de formule générale 1, caractérisé en ce qu'on fait réagir des 1,2-diphényléthanones de formule générale

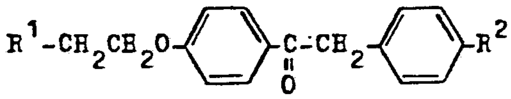

(2)

où $R^1$ a la signification donnée dans la formule générale 1 et $R^2$ représente un groupe méthoxy, dans le diméthylformamide anhydre avec de l'hydrure de sodium, après réaction avec du 1-chloro-1-phénylpropane en ce qu'on isole par cristallisation la forme érythro et le cas échéant en ce qu'on libère le groupe hydroxy par séparation sélective du groupe méthoxy avec de l'acide bromhydrique.

3. Médicament contenant un composé selon la revendication 1 comme substance active ainsi que ses supports et additifs habituels.